# EUROPEAN PATENT SPECIFICATION

(11) **EP 3 442 428 B1**
(45) Date of publication and mention of the grant of the patent: **02.03.2022**
(21) Application number: 17782044.6
(22) Date of filing: 22.03.2017
(51) Int. Cl.: A61B 10/00, G01N 33/53, G01N 33/68

(54) **ONE STEP CARDIAC TEST DEVICE**
EINSTUFIGE HERZTESTVORRICHTUNG
DISPOSITIF DE TEST CARDIAQUE À UNE ÉTAPE

(30) Priority: 13.04.2016 IL 24509116
(43) Date of publication of application: 20.02.2019
(73) Proprietor: NOVAMED LTD., 9777518 Jerusalem (IL)
(72) Inventor: KATZ, Emil, 5654822 Savyon (IL); TZUBERY, Tzvi, 9062700 Ofra (IL)
(74) Representative: Cabinet Becker et Associés
(86) International application number: PCT/IL2017/050359
(87) International publication number: WO 2017/179036

(56) References cited:
- EP-A1- 1 998 178
- WO-A1-2006/120391
- WO-A1-2011/113905
- WO-A1-2015/176589
- WO-A2-2008/066997
- CN-A- 104 714 031
- US-A1- 2011 076 692
- BODY, RICHARD ET AL.: 'A FABP-ulous 'rule out' strategy? Heart fatty acid binding protein and troponin for rapid exclusion of acute myocardial infarction' RESUSCITATION vol. 82, no. 8, 11 May 2011, pages 1041 - 1046, XP028240007

## Description

### Field of the Invention

The present invention relates to an immunoassay for detecting cardiac markers during early stages of myocardial infarction. Particularly, the invention provides a device for detecting diagnostically relevant combination of two blood markers as early as half an hour upon an acute myocardial infarction symptoms onset.

### Background of the Invention

About one million people have an acute myocardial infarction (AMI) each year in the United States alone, of whom 10% may die within one year, depending on the severity of the event and the quality of intervention. In any case, medical assistance, including primary percutaneous coronary intervention and/or thrombolytic therapy if needed, should be provided as promptly as possible. Shortening the duration between chest pain onset and reperfusion to less than about four hours was shown to be critical in reducing myocardial necrosis and improving heart function lowering 30-day mortality. [Ho Y.C. et al.: Crit. Care Med. 42 (2014) 1788-96]. Early diagnosis of the AMI is important, but the sensitivity of the classic ECG for diagnosing AMI in the patients presenting with ischemic-type chest pain is not much more than 50% [The Merck Manual, 17th Ed. (1999), p. 1673]. Blood cardiac markers are therefore of immense importance. Various proteins that leak out of injured myocardial cells into the bloodstream have served for assessing cardiac injury, including myoglobin or the enzymes like aspartate transaminase, lactate dehydrogenase, or creatine phosphokinase. Nowadays, it is mainly troponins which have been acknowledged as being enough specific for detecting myocardial damage. Unfortunately, cardiac troponins, although remaining elevated for up to 2 weeks after the AMI event, do not appear immediately and are well measurable only several hours after the AMI event, so that the first moments, when the correct diagnosis is most critical, are not well covered. It is therefore an object of this invention to provide a method for diagnosing acute myocardial infarction by detecting suitable blood markers. It has been found that heart-type fatty acid binding protein (HFBP) is another AMI marker, and conveniently the protein appears in the blood within about one hour and peaks within about five hours within the AMI event [Tanaka T. et al.: Clinical Biochemistry 24 (1991) 195-201; Van Nieuwenhoven F.A. et al.: Circulation 92 (1995) 2848-54], but the diagnostic accuracy of HFBP as a sole marker was found to be not sufficient in the clinical setting [Park S.Y. et al.: Clin. Neurol. Neurosurg. 115 (2013) 405-10]. It is another object of this invention to provide a method for diagnosing acute myocardial infarction by detecting more than one blood marker.

It is still another object of this invention to provide a device for diagnosing AMI which employs more than one blood marker of myocardial infarction.

It is a further object of this invention to provide a device for assisting in diagnosing AMI within the period of several hours after the AMI event.

Other objects and advantages of present invention will appear as description proceeds.

### Summary of the Invention

The present invention provides a device for detecting acute myocardial infarction (AMI) markers in a human blood sample, the device comprising a mixture of at least two antibodies or antibody conjugates which reacts with at least cardiac troponin (CT) and with heart-type fatty acids binding protein (HFBP). Said markers comprise CT and HFBP, wherein said antibody conjugates simultaneously react with said markers present in said blood sample, thereby providing a visible signal if the concentration of each of said markers is higher than a certain predetermined threshold. The antibodies, or antibody conjugates, react with said blood markers only after separating the blood plasma from the blood cells; the blood cells do not contact said antibodies as is known to an expert, as the blood cells are first removed on a prefilter; usually the plasma migrates toward the antibody or the antibody conjugate to form a marker-antibody complex or a marker-antibody conjugate complex, which provides a visible signal either by itself or after reacting with additional reagents. The threshold for each marker is derived from the distribution of its blood concentration in healthy population. In one embodiment of the invention, said threshold is higher than the 95^{th} percentile in said distribution for each of the markers. In another embodiment of the invention, said threshold is higher than the 99^{th} percentile in said distribution for each of the markers. In a preferred embodiment of the invention, said threshold for each marker is lower than the maximal concentration of the marker in the blood which does not provide a visible signal during the reaction with said antibodies or antibody conjugates, if said marker is alone in the sample. In one embodiment, said threshold for CT is 0.5 ng/ml and said threshold for HFBP is 5 ng/ml in the device according to the invention. In an example, said threshold for CT is about 1 ng/ml and said threshold for HFBP is about 10 ng/ml. In one embodiment, the device of the invention provides a visible signal if the concentration of CT in said blood sample is at least 0.5 ng/ml and simultaneously the concentration of HFBP in said blood sample is at least 5 ng/ml. In an example, the device of the invention provides a visible signal if the concentrations of CT and HFBP in said blood sample are at least 1 ng/ml and 5 ng/ml, respectively. In a still other example, the device of the invention provides a visible signal if the concentrations of CT and HFBP in said blood sample are at least 1 ng/ml and 10 ng/ml, respectively. Said troponin may comprise proteins selected from troponin-C, troponin-I, troponin-T, a complex of said troponins, or a mixture comprising at least two of said troponins. Preferably, troponin-I or troponin-T is employed as a cardiac marker. Said mixture of antibodies or antibody conjugates is usually immobilized in an immunoassay strip, in the device of the invention. Said strip may be a part of an immunoassay device, for example of a lateral flow immunoassay kit. In one embodiment, said immunoassay device comprises ELISA. Said mixture of antibodies or antibody conjugates is immobilized in an immunoassay strip as one narrow band, which is configured to change color in the presence of the antigens, namely of said AMI blood markers. Said visible signal is thus colorchanging of said band, caused by a color forming reaction of the marker with an antibody conjugate. In another aspect of the invention, said mixture of antibodies or antibody conjugates may be immobilized in said strip as several narrow bands differing in said predetermined threshold and providing visible signals at different concentrations of said markers (differing in sensitivity). The device of the invention may advantageously comprise reference bands, exhibiting a comparative color intensity either before or after contacting with said blood sample. The comparative bands may comprise antibodies or antibody conjugates reacting with typical blood proteins (albumins, immunoglobulins, etc.), the antibodies or antibody conjugates being conjugated in such a way and immobilized in such an amount so as to provide a color signal of the desired intensity in contact with said blood sample. The comparative bands may comprise a color of the desired intensity present in the device before contacting said sample and stable also after contacting said sample. Said color of the desired intensity may correspond to the expected color signal provided by relevant markers concentrations in the blood sample. A set of comparative bands enables to assess the concentration of the cardiac markers in the blood sample, when comparing a visible signal provided by a blood cardiac marker or a combination of markers with the set of comparative bands.

In a preferred embodiment of the invention, provided is a device for detecting an AMI event which comprises one or more antibody mixtures simultaneously specific both to CT and HFBP, the antibodies or antibody conjugates being immobilized in an immunoassay strip as one or several narrow bands and reacting with cardiac markers CT and HFBP in at least one blood sample to provide visible signal(s) if the concentration of each of said markers is higher than a certain predetermined threshold, said strip further comprising reference bands exhibiting a comparative color intensities either before or after contacting with said blood sample, wherein said several narrow bands optionally react with said markers in said blood sample with different sensitivities and provide said visible signals at different marker concentrations. The device of the invention provides an assessment of the CT and/or HFBP concentration in said sample, based either on comparing the intensities of said signal(s) with said comparative intensities or based on comparing two signals corresponding to two different marker concentrations. In one aspect of the invention, the assessment of the CT and/or HFBP concentration in said sample provides an assessment of the time elapsed between taking the sample and the AMI event. In one example of the device according to the invention, said predetermined threshold is 0.5 ng/ml for CT and 10 ng/ml for HFBP for one of said several narrow bands, wherein a positive signal indicates that an AMI event occurred in the donor of said sample at last one hour ago. In another example of the device said predetermined threshold is 1 ng/ml for CT and 20 ng/ml for HFBP for one of said several narrow bands, wherein a positive signal indicates that an AMI event occurred in the donor of said sample at last three hours ago. In other example of the device said predetermined threshold is 4 ng/ml for CT and 80 ng/ml for HFBP for one of said several narrow bands, wherein a positive signal indicates that an AMI event occurred in the donor of said sample at last nine hours ago. Combined signals of several "well-tuned" bands enables more accurate assessments. For example, the device may comprise a band with a threshold of 0.5 ng/ml for CT and 10 ng/ml for HFBP, and another band with a threshold of 1 ng/ml for CT and 20 ng/ml for HFBP, wherein a positive signal in the former band together with a negative signal in the latter band indicate that an AMI event occurred in the donor of said sample at a time between about 3 and about 6 hours ago.

Here provided is a method of diagnosing cardiac event, particularly an AMI, comprising taking a blood sample of a subject suspected of such event, for example a subject suffering from ischemic-type chest pain, and applying the sample on an immunoassay device comprising a mixture of at least two antibodies or antibody conjugates which reacts with at least CT and HFBP. In another aspect of the invention, at least two samples are taken at two different times, wherein concentration assessments of the CT and/or HFBP in said two sample improves the accuracy in assessing the time of the AMI event.

### Detailed Description of the Invention

The inventors found that simultaneous detection of two cardiac blood markers by employing a mixture of antibodies or antibody conjugates may obviate drawbacks of the previous methods aiming at early confirmation of a cardiac event in case of, for example, ischemic-type chest pain or other symptoms. The invention provides a one-step cardiac test device which enables a rapid diagnostic assessment in a subject suspected of an acute myocardial infarction (AMI), within the most critical period of several hours from the event. In one aspect, the invention provides a qualitative lateral flow immune chromatographic assay comprising a mixture of two antibodies or antibody conjugates, each of them specific to one cardiac marker, aiming at simultaneous detection of both markers at low concentrations characteristic for the early stages of the cardiac event, while the combined immunoassay signal provided by both markers, surpassing certain threshold level, indicates the event. One of the markers is a cardiac troponin (CT) and the other is heart-type fatty acid binding protein (HFBP). In one example, HFBP is detected about half an hour upon symptoms onset while, of which levels at that time is about 5 ng/ml (basal levels in 95% of the healthy population are in the range of 0.5-3.5), whereas troponin-I becames detectable at about three hours after the symptoms, when its levels are above 1 ng/ml. For example, clinical results showed the additive effect of two markers, wherein each one alone was undetectable below specific concentration (usually 1ng/ml for troponin and 5 ng/ml for HFBP), but both of them together in the same concentrations provided true positive results; in one example, 0.14 ng/ml troponin or 4.92 ng/ml FABP was undetectable, but a sample containing 0.14 ng/ml troponin with 4.92 ng/ml HFBP became detectable.

When relating to "antibody" in the description of the invention, the relation may be to antibody, as well as to antibody derivative or antibody conjugate, the relevant aspect being the ability of the agent to react with the antigen, namely with the cardiac marker. The mixture of antibodies in a device or method according to the invention may comprise more than one antibody against one marker; the mixture may comprise antibodies against several forms of the marker, such as against several forms of troponin, or against several epitopes on the same marker. Of course, that the device and method according to the invention employs reactions known in the art, such as reactions of antibody-antigen complex with another antibody or antibody conjugate, the aim being as strong visible signal as needed. In one embodiment of the invention, for example, the device comprises two antibodies for the reaction with CT and one antibody for the reaction with HFBP, and furher two antibody conjugates for reacting with the CT complexes and one antibody conjugate for reacting with the HFBP complexes.

In one aspect, the assay aims at qualitative determination of HFBP and troponin I in a sample of the whole blood of a diagnosed subject, said markers usually detectable in blood within 1 hour and within 4 hours, respectively, after the myocardial damage, when checked separately, wherein it should be noted that current practice does not rely on the detection of HFBP alone.

Means for separately detecting HFBP and troponin I have been described; however the invention combines two separate reactions into one effect resulting in one indicative signal. In an important aspect of the invention, a device comprising a mixture of antibodies or antibody conjugates specific to two cardiac markers simultaneously detects combined minimal levels of cardiac troponin I and/or HFBP; in one embodiment of the invention, said minimal levels may be hardly detectable when measured separately. Advantageously, the device of the invention provides results in few minutes, for example 10 to 15 minutes; very importantly, the results may be obtained within the first several most critical hours from an cardiac event, such as within four hours, for example within three hours or within two hours. The fact is that a positive result in detecting increased levels of any one of HFBP and CT after a suspected cardiac event in a subject indicates an emergency situation and directs the subject to the hospital; the novel attitude of combining both markers to a single line simplifies the interpretation of the actionable results. Moreover, the common test line for two cardiac markers improves the sensitivity of the test, in which the complexes of more antibodies or antibody conjugates, such as anti-HFBP and anti-troponin I, contribute to the intensity of the same band. Thanks to this accumulation effect, lower markers amounts are detectable in one band comprising an antibody mixture than in two separate bands comprising separate antibodies or antibody conjugates. However, the main advantage of the new device and method is an averaging effect of the signals combination in the critical period of between 1 and 4 hours from an AMI event, which provides rapid and reliable results nearly online.

The invention will be further described and illustrated in the following examples.

### Examples

Antibody conjugates providing color reaction with CT, HFBP, or human serum albumin were obtained, particularly anti-troponin I, anti-HFBP, and anti-HSA. The antibody conjugates were employed for immobilization, in narrow bands, on strips of capillary beds configured to immobilize antibody conjugates. The strips were employed in preparing lateral flow immunoassay devices (LFID). By immobilizing a series of conjugate quantities/ concentrations (volumes of certain concentrations to be applied onto the strip for the conjugate immobilization corresponding to different total amounts of color-forming reagents in the band), and by reacting the bands with a series of samples containing different antigen concentrations, the needed reagent quantities for desired signal intensities were determined for all antigens. The following threshold quantities/concentrations were determined: the quantity of anti-troponin I for detecting 1 ng/ml troponin I in the sample, and the quantity of anti-FABP-3 for detecting 5 ng/ml HFBP in the sample, the threshold quantities corresponding to the maximal quantity providing an imperceptible color signal. Samples comprising either 3 ng/ml troponin-I or 15 mg/ml HFBP provided under the same conditions quite clear color bands. Anti-HAS was employed in such a quantity, so as to provide a strong signal (about 10 times stronger than 3 ng/ml troponin-I with the troponin band) and serve as a control that the LFID principally works. A mixture of antibody conjugates was prepared by mixing said threshold quantity of anti-troponin I and said threshold quantity of FABP-3.

Nine LFIDs were prepared, all comprising a control HAS band; three comprised threshold quantity for detecting 1 ng/ml troponin-I, three comprised threshold quantity for detecting 5 ng/ml HFBP, and three comprising the band with mixed antibody conjugates comprising said threshold quantities of both anti-troponin-I and anti-HFBP. A sample of human blood from a healthy donor (very low levels of the cardiac markers) was divided to three portions: troponin-I was added to the first portion to a concentration of 1 ng/ml, HFBP was added to the second portion to a concentration of 5 ng/ml, and both markers were added in concentrations of 1 and 5 ng/ml, respectively, in the third portion. Each portion was applied on the LFIDs comprising relevant antibody. Perceptible signals appeared (+) or did not appear (-) in the devices as shown in Table 1.

**Table 1**

| The presence (+) or absence (-) of a visible signal in nine devices. Three devices comprised anti-CT, three devices anto-HFBP, and three the antibody mixture; a blood sample comprising 1 ng/ml of added troponin-I was applied on the anti-CT devices; a blood sample comprising 5 ng/ml of HFBP was applied on the anti-HFBP devices; a blood sample comprising 1 ng/ml of added troponin-I and 5 ng/ml of added HFBP was applied on the mixture devices. C stands for control, M stands for cardiac marker. | | | | | | | | | |
|---|---|---|---|---|---|---|---|---|---|
| | anti-CT | | | anti-HFBP | | | anti-CT+ anti-HFBP | | |
| C | + | + | + | + | + | + | + | + | + |
| M | - | - | - | - | - | - | + | + | + |

The table shows consistent results provided by the devices (triplicate). The concentrations of 1 and 5 ng/ml of the two markers, respectively, correspond to a very early stage of the cardiac event. The table shows that a device according to the invention can provide relevant information contributing to early diagnosing an AMI.

While this invention has been described in terms of some specific examples, many modifications and variations are possible. It is therefore understood that within the scope of the appended claims, the invention may be realized otherwise than as specifically described.

## Claims

1. A device for detecting acute myocardial infarction (AMI) markers in a human blood sample, comprising a mixture of two antibodies or antibody conjugates, each of them specific to one cardiac marker, which are immobilized in an immunoassay strip in a band to react with both cardiac troponin (CT) at a threshold concentration of 0.5 ng/ml and heart-type fatty acids binding protein (HFBP) at a threshold concentration of 5 ng/ml.

2. The device of claim 1, wherein said threshold for each marker is derived from the distribution of its blood concentration in healthy population.

3. The device of claim 2, wherein said threshold is higher than the 95^{th} percentile, such as the 99^{th} percentile, in said distribution for each of the markers.

4. The device of claim 2, wherein said threshold for each marker is lower than the maximal concentration of the marker which does not provide a visible signal if said marker is alone in the sample.

5. The device of claim 1, wherein said troponin comprises proteins selected from the group consisting of troponin-C, troponin-I, troponin-T, a complex of said troponins, and a mixture comprising at least two of said troponins.

6. The device of claim 1, wherein said immunoassay strip is a part of a lateral flow immunoassay kit.

7. The device of claim 6, wherein said mixture of antibodies or antibody conjugates is immobilized in said strip in narrow bands differing in said threshold and providing said visible signal at different concentrations of said markers.

8. The device of claim 1, comprising ELISA.

9. The device of claim 1, comprising one or more mixtures of antibodies or antibody conjugates, said mixtures simultaneously specific both to CT and HFBP, the antibodies or antibody conjugates being immobilized in an immunoassay strip as one or several narrow bands and reacting with said markers in at least one blood sample to provide visible signal(s) if the concentration of each of said markers is higher than a certain predetermined threshold, said strip further comprising reference bands exhibiting a comparative color intensities either before or after contacting with said blood sample, wherein said several narrow bands optionally react with said markers in said blood sample with different sensitivities and providing said visible signals at different marker concentrations.

10. The device of claim 9, providing an assessment of the CT and/or HFBP concentration in said sample, based either on comparing the intensities of said signal(s) with said comparative intensities or on comparing two signals corresponding to two different marker concentrations, wherein said assessment optionally provides an estimation of the time elapsed between taking the sample and the AMI event.

11. The device of claim 9, wherein said predetermined threshold is 0.5 ng/ml for CT and 10 ng/ml for HFBP for one of said several narrow bands, and wherein a positive signal indicates that an AMI event occurred in the donor of said sample at last one hour ago.

12. The device of claim 9, wherein said at least one blood sample comprises at least two samples taken at two different times, and wherein concentration assessments of the CT and/or HFBP concentrations in said two sample improves the accuracy in assessing the time of the AMI event.

## Patentansprüche

1. Vorrichtung zum Nachweis von akuten Myokardinfarkt(AMI)-Markern in einer menschlichen Blutprobe, umfassend eine Mischung aus zwei Antikörpern oder Antikörper-Konjugaten, die jeweils spezifisch für einen Herzmarker sind, die in einem Immunoassay-Streifen in einer Bande immobilisiert sind, zur Reaktion sowohl mit kardialem Troponin (CT; cardiac troponin) bei einer Schwellenkonzentration von 0,5 ng/ml als auch mit fettsäurebindendem Protein vom Herztyp (HFBP; heart-type fatty acids binding protein) bei einer Schwellenkonzentration von 5 ng/ml.

2. Vorrichtung nach Anspruch 1, wobei dieser Schwellenwert für jeden Marker aus der Verteilung seiner Blutkonzentration in einer gesunden Bevölkerung abgeleitet wird.

3. Vorrichtung nach Anspruch 2, wobei der Schwellenwert in der Verteilung für jeden der Marker höher ist als das 95. Perzentil, beispielsweise als das 99. Perzentil.

4. Vorrichtung nach Anspruch 2, wobei der Schwellenwert für jeden Marker niedriger ist als die maximale Konzentration des Markers, der kein sichtbares Signal liefert, wenn der Marker allein in der Probe ist

5. Vorrichtung nach Anspruch 1, wobei das Troponin Proteine umfasst, ausgewählt aus der Gruppe bestehend aus Troponin-C, Troponin-I, Troponin-T, einem Komplex dieser Troponine und einer Mischung, die mindestens zwei dieser Troponine umfasst.

6. Vorrichtung nach Anspruch 1, wobei der Immunoassay-Streifen ein Teil eines Lateral-Flow-Immunoassay-Kits ist.

7. Vorrichtung nach Anspruch 6, wobei die Mischung von Antikörpern oder Antikörperkonjugaten in schmalen Banden immobilisiert ist, die sich in dieser Schwelle unterscheiden und das sichtbare Signal bei unterschiedlichen Konzentrationen der Marker bereitstellen.

8. Vorrichtung nach Anspruch 1, umfassend ELISA.

9. Vorrichtung nach Anspruch 1, umfassend eine oder mehrere Mischungen von Antikörpern oder Antikörper-Konjugaten, wobei diese Mischungen gleichzeitig sowohl für CT als auch HFBP spezifisch sind, wobei die Antikörper oder Antikörper-Konjugate in einem Immunoassay-Streifen als eine od er mehrere schmale Banden immobilisiert sind und mit den genannten Marker in mindestens einer Blutprobe, um sichtbare Signale bereitzustellen, wenn die Konzentration jedes der Marker höher als ein bestimmter vorbestimmter Schwellenwert ist, wobei dieser Streifen ferner Referenzbanden umfasst, die entweder vor oder nach dem Kontaktieren mit der Blutprobe eine vergleichbare Farbintensität aufweisen, wobei diese mehreren schmalen Banden optional mit den Markern in der Blutprobe mit unterschiedlichen Empfindlichkeiten reagieren und die sichtbaren Signale bei unterschiedlichen Markerkonzentrationen bereitstellen

10. Vorrichtung nach Anspruch 9, die eine Bewertung der CT- und/oder HFBP-Konzentration in der Probe bereitstellt, basierend entweder auf dem Vergleich der Intensitäten dieses Signals/dieser Signale mit den Vergleichsintensitäten oder auf dem Vergleich von zwei Signalen, die zwei unterschiedlichen Markerkonzentrationen entsprechen, wobei diese Bewertung optional eine Schätzung der zwischen der Entnahme der Probe und dem AMI-Ereignis verstrichenen Zeit bereitstellt

11. Vorrichtung nach Anspruch 9, wobei der vorbestimmte Schwellenwert 0,5 ng/ml für CT und 10 ng/ml für HFBP für eines dieser mehreren schmalen Bänder beträgt, und wobei ein positives Signal anzeigt, dass ein AMI-Ereignis im Spender dieser Probe vor wenigstens einer Stunde aufgetreten ist.

12. Vorrichtung nach Anspruch 9, wobei diese mindestens eine Blutprobe mindestens zwei Proben umfasst, die zu zwei unterschiedlichen Zeitpunkten entnommen wurden, und wobei Konzentrationsbewertungen der CT-und/oder HFBP-Konzentrationen in diesen beiden Proben die Genauigkeit bei der Bewertung des Zeitpunkts des AMI-Ereignisses verbessert.

## Revendications

1. Dispositif pour détecter des marqueurs d'infarctus du myocarde aigu (IMA) dans un échantillon de sang humain, comprenant un mélange de deux anticorps ou conjugués d'anticorps, chacun d'eux spécifique d'un marqueur cardiaque donné, qui sont immobilisés dans une bandelette d'immunodosage dans une bande pour réagir avec tant la troponine cardiaque (CT) à une concentration seuil de 0,5 ng/ml et qu'avec la protéine se liant à des acides gras de type cardiaque (HFBP) à une concentration seuil de 5 ng/ml.

2. Dispositif selon la revendication 1, dans lequel ledit seuil pour chaque marqueur dérive de la distribution de sa concentration sanguine dans la population en bonne santé.

3. Dispositif selon la revendication 2, dans lequel ledit seuil est supérieur au 95ème percentile, tel que le 99ème percentile, dans ladite distribution pour chacun des marqueurs.

4. Dispositif selon la revendication 2, dans lequel ledit seuil pour chaque marqueur est inférieur à la concentration maximale du marqueur qui ne donne pas un signal visible si ledit marqueur est seul dans l'échantillon.

5. Dispositif selon la revendication 1, dans lequel ladite troponine comprend des protéines choisies dans le groupe constitué par la troponine C, la troponine I, la troponine T, un complexe desdites troponines, et un mélange comprenant au moins deux desdites troponines.

6. Dispositif selon la revendication 1, dans lequel ladite bandelette d'immunodosage fait partie d'une trousse d'immunodosage à écoulement latéral.

7. Dispositif selon la revendication 6, dans lequel ledit mélange d'anticorps ou de conjugués d'anticorps est immobilisé dans ladite bandelette en bandes étroites différant par ledit seuil et donnant ledit signal visible à différentes concentrations desdites marqueurs.

8. Dispositif selon la revendication 1, comprenant un ELISA.

9. Dispositif selon la revendication 1, comprenant un ou plusieurs mélanges d'anticorps ou de conjugués d'anticorps, lesdits mélanges étant simultanément spécifiques tant de CT que de HFBP, les anticorps ou conjugués d'anticorps étant immobilisés dans une bandelette d'immunodosage sous la forme d'une ou de plusieurs bandes étroites et réagissant avec lesdits marqueurs dans au moins un échantillon de sang pour donner un ou plusieurs signaux visibles si la concentration de chacun desdits marqueurs est supérieure à un certain seuil prédéterminé, ladite bandelette comprenant en outre des bandes de référence présentant des intensités de couleur comparatives soit avant soit après mise en contact avec ledit échantillon de sang, dans lequel lesdites plusieurs bandes étroites réagissent éventuellement avec lesdits marqueurs dans ledit échantillon de sang avec des sensibilisés différentes et donnant lesdits signaux visibles à différentes concentrations de marqueurs.

10. Dispositif selon la revendication 9, donnant une détermination de la concentration de CT et/ou HFBP dans ledit échantillon, sur la base soit d'une comparaison des intensités dudit ou desdits signaux avec lesdites intensités comparatives, soit d'une comparaison de deux signaux correspondant à deux concentrations de marqueurs différentes, dans lequel ladite détermination donne éventuellement une estimation du temps écoulé entre la prise de l'échantillon et l'événement IMA.

11. Dispositif selon la revendication 9, dans lequel ledit seuil prédéterminé est de 0,5 ng/ml pour la CT et de 10 ng/ml pour la HFBP pour l'une parmi lesdites plusieurs bandes étroites, et dans lequel un signal positif indique qu'un événement IMA est survenu chez le donneur dudit échantillon au final une heure plus tôt.

12. Dispositif selon la revendication 9, dans lequel ledit au moins un échantillon de sang comprend au moins deux échantillons pris à deux moments différents, et dans lequel les déterminations de concentration des concentrations de CT et/ou HFBP dans lesdits deux échantillons améliorent la précision de la détermination du moment de l'événement IMA.
